# EUROPEAN PATENT APPLICATION

(11) **EP 2 163 246 A1**
(43) Date of publication of application: **17.03.2010**
(21) Application number: 08305546.7
(22) Date of filing: 12.09.2008
(51) Int. Cl.: A61K 31/185, A61P 43/00

(54) **Taurine or taurine-like substances for the prevention of the irreversible visual effects of vigabatrin**

(71) Applicant: INSERM (Institut National de la Santé et de la Recherche Médicale), 75654 Paris Cedex 13 (FR)
(72) Inventor: Picaud, Serge, 75012 Paris (FR); Sahel, José Alain, 75012 Paris (FR); Jammoul, Firas, 75012 Paris (FR); Coriat, Caroline, 75012 Paris (FR); Simonutti, Manuel, 75012 Paris (FR)
(74) Representative: Hirsch, Denise Marie

(57) **Abstract**

The present invention relates to taurine or taurine-like substances for the prevention of the irreversible visual effects of vigabatrin. More particularly the invention relates to a substance selected from the group consisting of taurine, a taurine precursor, a taurine metabolite, a taurine derivative, a taurine analog and a substance required for the taurine biosynthesis for the prevention of the irreversible visual effects of vigabatrin.

## Description

### FIELD OF THE INVENTION:

The prevention invention relates to taurine or taurine-like substances for the prevention of the irreversible visual effects of vigabatrin.

### BACKGROUND OF THE INVENTION:

A deficiency of GABA in the brain has been implicated as one cause for convulsions. (Karlsson, A.; Funnum, F.; Malthe-Sorrensen, D.; Storm-Mathisen, J. Biochem Pharmacol 1974, 22, 3053-3061). To correct the deficiency of brain GABA and therefore stop convulsions, an important approach is to use an inhibitor of GABA-aminotransferase (GABA-AT) that is able to cross blood-brain barrier. (Nanavati, S. M.; Silverman, R. B. J. Med. Chem. 1989, 32, 2413-2421.). Inhibition of this enzyme increases the concentration of GABA in the brain, which has therapeutic applications in epilepsy as well as other neurological disorders. One of the most effective in vivo time-dependent inhibitors of GABA-AT is 4-amino-5-hexenoic acid, which is also termed gamma-vinyl GABA or vigabatrin, an anticonvulsant drug marketed almost all over the world.

However, vigabatrin has been shown to induce highly severe undesirable effects. There have been several reports of altered visual acuity (Jonhson MA et al. 2000), colour discrimination (Hilton EJ. et al. 2002) or contrast sensitivity (Hilton EJ. et al. 2002; Miller NR. et al. 1999) whereas other studies, such as the longitudinal cohort studies sponsored by the National Health Service of Scotland, have demonstrated preservation of central and colour vision in patients with bilateral constriction (McDonagh J. et alL 2003). In children treated for infantile spasm, dysfunction of retinal ganglion cells was attested by retinal nerve fiber atrophy (Bunsic et al., 2004). The degeneration of retinal ganglion cells was demonstrated on histological section of the retina from an adult vigabatrin-treated patient (Ravindran et al., 2001).

In rats, vigabatrin accumulates in the retina at a concentration five-fold higher than in the brain (Sills GJ. et al. 2001). Consequently, GABA concentrations increase five-fold higher in the retina and two-fold higher in the brain than those of control rats (Neal MJ. et al. 1989; Cubells JF. et al. 1987). Vigabatrin treatment disorganises the photoreceptor layer in the peripheral retina (Butler WH. et al. 1987; Duboc A. et al. 2004; Wang QP. et al. 2008), damages cone photoreceptors in broader areas (Duboc A. et al. 2004; Wang QP. et al. 2008) and reduces the photopic ERG and flicker responses (Duboc A. et al. 2004). The lesion area is marked by major glial reaction, and can be visualised by immunohistochemical staining for the glial fibrillary acidic protein (GFAP) (Duboc a. et al. 2004), a hallmark of retinal lesions. In vitro, vigabatrin-elicited photoreceptor degeneration was reported to be light-dependent (Izumi Y. et al. 2004). This is consistent with the selective vigabatrin sensitivity of albino but not pigmented animals (Butler WH. et al. 1987).

Despite these irreversible visual effects, Vigabatrin remains in infantile spasms the only alternative to adrenocorticotrophic hormone (ACTH) or steroid therapy (Ben-Menachem E. et al. 2008; Chiron C. et al. 1997; Lux Al. et al. 2005; Dulac O. et al. 2008; Snead OC. Et al. 1983; Hrachovy RA. et al. 1994; Baram TZ. et al. 1996). It is also prescribed as a third-line drug for other refractory epilepsies in Europe (Ben-Menachem E. et al. 2008). Furthermore, it is being evaluated for treatment of heroin, cocaine and methamphetamine addictions (Gerasimov MR. et al. 1999; Stromberg MF. et al. 2001).

There is thus a need in the art for substances that would allow preventing the irreversible visual effects of vigabatrin.

### SUMMARY OF THE INVENTION:

The present invention relates to a substance selected from the group consisting of taurine, a taurine precursor, a taurine metabolite, a taurine derivative, a taurine analog and a substance required for the taurine biosynthesis for the prevention of the irreversible visual effects of vigabatrin.

The present invention also relates to a pharmaceutical composition for the prevention of the irreversible visual effects of vigabatrin which comprises a substance as above described and optionally one or more pharmaceutically acceptable excipients.

Finally, the present invention relates to a pharmaceutical composition for the treatment of convulsive disorders which comprises (i) a first active ingredient which is vigabatrin; and (ii) a second active ingredient selected from the group consisting of taurine, a taurine precursor, a taurine metabolite, a taurine derivative, a taurine analog and a substance required for the taurine biosynthesis and (iii) optionally one or more pharmaceutically acceptable excipients.

### DETAILED DESCRIPTION OF THE INVENTION:

### Definitions:

The term "vigabatrin" refers to 4-amino-5-hexenoic acid which has the formula of : 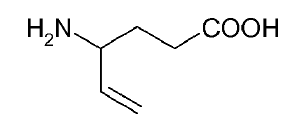 The term encompasses the racemic mixture of vigabatrin or the active isomer.

The term "taurine" refers to 2-aminoethanesulfonic acid which has the formula of:

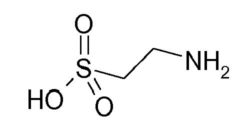

As used herein, "taurine precursors" encompass substances that, when they are administered to a human or an animal, can be transformed, directly or indirectly, into taurine.

As used herein, "taurine metabolites" encompass substances that are produced in vivo by transformation of taurine.

As used herein, "taurine derivatives" encompass substances that are structurally close to taurine but possess at least one structural difference, such as one or more chemical changes, e.g. at least one replacement of an atom or a chemical group found in taurine by a distinct atom or a distinct chemical group.

As used herein, "taurine analogs" encompass substances that are chemically distinct from taurine but which exert the same biological activity.

As used herein, "substances required for taurine biosynthesis" encompass all substances that are involved in the in vivo taurine biosynthesis including enzymes and enzyme cofactors, thus including cysteine dioxygenase (EC 1.13.11), sulfinoalanine decarboxylase (EC 4.1.1.29) and cofactors thereof.

As intended herein, taurine precursors, taurine metabolites, taurine derivatives, taurine analogs and substances required for the taurine biosynthesis may be collectively termed "taurine-like substances".

The term "irreversible visual effects" denotes all the side effects that can be induced by vigabatrin in the eye, such as irreversible constriction of the visual field, dysfunction of central vision with a reduction of visual acuity, and a loss of color discrimination and of contrast sensitivity. More particularly the irreversible visual effects of vigabatrin includes but is not limited to a decrease in the photopic electroretinogram (ERG) measurement, photoreceptor degeneration, retinal disorganisation, sprouting of bipolar cell dendrites and degeneration of retinal ganglion cells.

According to the invention, the term "subject" or "patient" and "subject in need thereof" or "patient in need thereof", is intended for a human or a non-human mammal that shall be treated with vigabatrin.

### Therapeutic methods and uses

Surprisingly, it has been found according to the invention that the progressive and irreversible loss of visual acuity of patients treated with the extracellular GABA inducer vigabatrin is associated with a high decrease in circulating taurine concentration. Notably, a taurine deficiency has been found in vigabatrin-treated epileptic infants. The inventors demonstrate accordingly that a supplementation of vigabatrin-treated individuals with taurine or with a substance required for the taurine biosynthesis inhibits the irreversible visual effects of vigabatrin.

Thus, an object of the present invention relates to a substance selected from the group consisting of taurine, a taurine precursor, a taurine metabolite, a taurine derivative, a taurine analog and a substance required for the taurine biosynthesis, for the prevention of the irreversible visual effects of vigabatrin.

An embodiment of the invention relates to a substance selected from the group consisting of taurine, a taurine precursor, a taurine metabolite, a taurine derivative, a taurine analog and a substance required for the taurine biosynthesis, for the prevention of the degeneration of photoreceptors induced by vigabatrin.

An embodiment of the invention relates to a substance selected from the group consisting of taurine, a taurine precursor, a taurine metabolite, a taurine derivative, a taurine analog and a substance required for the taurine biosynthesis, for the prevention of the degeneration of retinal ganglion cells induced by vigabatrin.

In a particular embodiment, taurine precursors are selected from the group consisting of cysteine, cystathionine, homocysteine, S-adenosylhomocysteine, serine, N-acetyl-cysteine, glutathione, N-formylmethionine, S-adenosylmethionine, betaine and methionine.

In another particular embodiment, taurine metabolites are selected from the group consisting of hypotaurine, thiotaurine, taurocholate.

In another particular embodiment, taurine derivatives are selected from different entities including the group consisting of acetylhomotaurinate, and piperidino-, benzamido-, phthalimido- or phenylsuccinylimido taurine derivatives. Such taurine derivatives are described notably by Kontro et al. (1983, Prog Clin Biol Res, Vol. 125 : 211-220) and by Andersen et al. (2006, Journal of pharmaceutical Sciences, Vol. 73(n°1): 106-108). Derivatives include for instance taurolidine (4,4'-methylene-bis(tetrahydro-2H-1,2,4-thiadiazine-1,1-dioxide or taurolin), taurultam and taurinamide, chlorohydrate-N-isopropylamide-2-(1-phenylethyl)aminoethanesulfonic acid.

In another particular embodiment taurine analogs are selected from the group consisting of (+/-)piperidine-3-sulfonic acid (PSA), 2-aminoethylphosphonic acid (AEP), (+/-)2-acetylaminocyclohexane sulfonic acid (ATAHS), 2-aminobenzenesulfonate (ANSA), hypotaurine,. ± trans-2-aminocyclopentanesulfonic acid (TAPS) 8-tétrahydroquinoléine sulfonic acid (THQS), N-2-hydroxyethylpiperazine-N'-2-ethane sulphonic acid (HEPES), beta-alanine, glycine, guanidinoethylsulfate (GES), 3-acetamido-1-propanesulfonic acid (acamprosate).

In another particular embodiment, substances required for taurine biosynthesis are selected from the group consisting of vitamin B6 (or pyridoxal-5'-phosphate), vitamin B12 (cobalamin), folic acid, riboflavin, pyridoxine, niacin, thiamine (thiamine pyrophosphate) and pantothenic acid.

This invention also relates to a therapeutic method for the prevention of the irreversible visual effects of vigabatrin, wherein method comprises a step of administering to a subject in need thereof with an effective amount of a substance selected from the group consisting of taurine, a taurine precursor, a taurine metabolite, a taurine derivative, a taurine analog and a substance required for the taurine biosynthesis.

An embodiment of the invention, relates to a therapeutic method for the prevention of the degeneration of photoreceptors induced by vigabatrin, wherein method comprises a step of administering to a subject in need thereof with an effective amount of a substance selected from the group consisting of taurine, a taurine precursor, a taurine metabolite, a taurine derivative, a taurine analog and a substance required for the taurine biosynthesis.

An embodiment of the invention, relates to a therapeutic method for the prevention of the degeneration of retinal ganglion cells induced by vigabatrin, wherein method comprises a step of administering to a subject in need thereof with an effective amount of a substance selected from the group consisting of taurine, a taurine precursor, a taurine metabolite, a taurine derivative, a taurine analog and a substance required for the taurine biosynthesis.

Generally speaking, a "therapeutically effective amount", or "effective amount", or "therapeutically effective", as used herein, refers to that amount which provides a therapeutic effect for a given condition and administration regimen. This is a predetermined quantity of active material calculated to produce a desired therapeutic effect in association with the required additive and diluent; i.e., a carrier, or administration vehicle. Further, it is intended to mean an amount sufficient to reduce and most preferably prevent a clinically significant deficit in the activity, function and response of the host. Alternatively, a therapeutically effective amount is sufficient to cause an improvement in a clinically significant condition in a host. As is appreciated by those skilled in the art, the amount of a compound may vary depending on its specific activity. Suitable dosage amounts may contain a predetermined quantity of active composition calculated to produce the desired therapeutic effect in association with the required diluents; i.e., carrier, or additive.

The present invention also relates to methods for the prevention or the treatment of convulsive disorders comprising a step of administering, to a subject in need thereof, a combination of (i) a first active ingredient which is vigabatrin; and (ii) a second active ingredient selected from the group consisting of taurine, a taurine precursor, a taurine metabolite, a taurine derivative, a taurine analog and a substance required for the taurine biosynthesis,

The subjects in need of such treatments encompass those, either adult or child patients, which are susceptible to various convulsive disorders including primarily convulsive disorders. Convulsive disorders encompass epilepsy, tuberous sclerosis, infantile spasms as well as the convulsive disorders affecting patients undergoing a drug addiction, including a drug addiction to heroin or cocaine, ethanol.

The first and the second active ingredients are comprised in an anticonvulsive pharmaceutical composition in a "therapeutically effective amount", that is in an amount sufficient for the combination of active ingredients to exert the expected anticonvulsive effect while inducing no irreversible visual effects, or a irreversible visual effects that is reduced as compared with pharmaceutical compositions comprising vigabatrin without taurine nor a taurine-like substance.

### Pharmaceutical compositions:

The present invention also pertains to pharmaceutical compositions comprising a substance selected from the group consisting of taurine, a taurine precursor, a taurine metabolite, a taurine derivative, a taurine analog and a substance required for the taurine biosynthesis, for the prevention of the irreversible visual effects of vigabatrin.

An embodiment of the invention relates to pharmaceutical compositions comprising a substance selected from the group consisting of taurine, a taurine precursor, a taurine metabolite, a taurine derivative, a taurine analog and a substance required for the taurine biosynthesis, for the prevention of the degeneration of photoreceptors induced by vigabatrin.

An embodiment of the invention relates to pharmaceutical compositions comprising a substance selected from the group consisting of taurine, a taurine precursor, a taurine metabolite, a taurine derivative, a taurine analog and a substance required for the taurine biosynthesis, for the prevention of the degeneration of retinal ganglion cells induced by vigabatrin.

In particular embodiment pharmaceutical compositions according to the invention comprise a first active ingredient which is vigabatrin and a second active active ingredient which is taurine or a taurine-like substance.

The pharmaceutical compositions according to the invention are suitable for treating various convulsive disorders including primarily convulsive disorders. Convulsive disorders encompass epilepsy, tuberous sclerosis, infantile spasms as well as the convulsive disorders affecting patients undergoing a drug addiction, including a drug addiction to heroin or cocaine, ethanol.

Thus, a pharmaceutical composition according to the invention consists primarily of an anti-convulsive pharmaceutical composition.

In a pharmaceutical composition according to the invention, the amount of the first active ingredient (vigabatrin) is the amount of the said active ingredient that is conventionally used for treating patients affected with convulsive disorders. Typically, the said pharmaceutical composition is adapted so that the dosage form used allows the administration of an amount of vigabatrin ranging between 10 µg and 10 grams per day, preferably between 100 µg and 5 grams, including between 1 mg and 1 gram, for a human adult patient having a mean weight of 80 kilos. Typically, a dosage form will contain half the daily dose, for the purpose of performing two takes per day. Lower amounts of vigabatrin may be used, especially when the active ingredient is not under the racemic form but instead under the form of its active isomer, which lower amounts are typically half the amount of the racemic form which would have been conventionally used.

In a pharmaceutical composition according to the invention, the amount of the second active ingredient, i.e. taurine or a taurine-like substance, is adapted so that the said pharmaceutical composition is adapted so that the dosage form used allows the administration of an amount of taurine or of the taurine-like substance ranging from 10 µg to 10 grams per day for a human adult patient having a mean weight of 80 kilos.

Indeed, in a pharmaceutical composition, the active ingredient(s) is (are) used in combination with one or more pharmaceutically or physiologically acceptable excipients.

Generally, a pharmaceutical composition according to the invention, irrespective of whether the said composition (i) comprises only one or more substances selected from taurine and taurine-like substances or (ii) comprises a combination of a first active ingredient which is vigabatrin and a second active ingredient selected from taurine and taurine-like substances, comprises the one or more active ingredients in an amount ranging from 0.1% to 99.9% by weight, and usually from 1% to 90% by weight, based on the total weight of the said pharmaceutical composition.

Generally, a pharmaceutical composition according to the invention comprises an amount of excipient(s) that ranges from 0.1% to 99.9% by weight, and usually from 10% to 99% by weight, based on the total weight of the said pharmaceutical composition.

By "physiologically acceptable excipient or carrier" is meant solid or liquid filler, diluents or substance which may be safely used in systemic or topical administration. Depending on the particular route of administration, a variety of pharmaceutically acceptable carriers well known in the art include solid or liquid fillers, diluents, hydrotropes, surface active agents, and encapsulating substances.

Pharmaceutically acceptable carriers for systemic administration that may be incorporated in the composition of the invention include sugar, starches, cellulose, vegetable oils, buffers, polyols and alginic acid. Specific pharmaceutically acceptable carriers are described in the following documents, all incorporated herein by reference: U.S. Pat. No. 4,401,663, Buckwalter et al. issued August 30, 1983; European Patent Application No. 089710, LaHann et al. published Sept. 28, 1983; and European Patent Application No. 0068592, Buckwalter et al. published Jan. 5, 1983. Preferred carriers for parenteral administration include propylene glycol, pyrrolidone, ethyl oleate, aqueous ethanol, and combinations thereof.

Representative carriers include acacia, agar, alginates, hydroxyalkylcellulose, hydroxypropyl methylcellulose, carboxymethylcellulose, carboxymethylcellulose sodium, carrageenan, powdered cellulose, guar gum, cholesterol, gelatin, gum agar, gum arabic, gum karaya, gum ghatti, locust bean gum, octoxynol 9, oleyl alcohol, pectin, poly(acrylic acid) and its homologs, polyethylene glycol, polyvinyl alcohol, polyacrylamide, sodium lauryl sulfate, poly(ethylene oxide), polyvinylpyrrolidone, glycol monostearate, propylene glycol monostearate, xanthan gum, tragacanth, sorbitan esters, stearyl alcohol, starch and its modifications. Suitable ranges vary from about 0.5% to about 1%.

For formulating a pharmaceutical composition according to the invention, the one skilled in the art will advantageously refer to the last edition of the European pharmacopoeia or of the United States pharmacopoeia.

Preferably, the one skilled in the art will refer to the fifth edition "2005" of the European Pharmacopoeia, or also to the edition USP 28-NF23 of the United States Pharmacopoeia.

The invention will be further illustrated by the following figures and examples. However, these examples and figures should not be interpreted in any way as limiting the scope of the present invention.

### FIGURES:

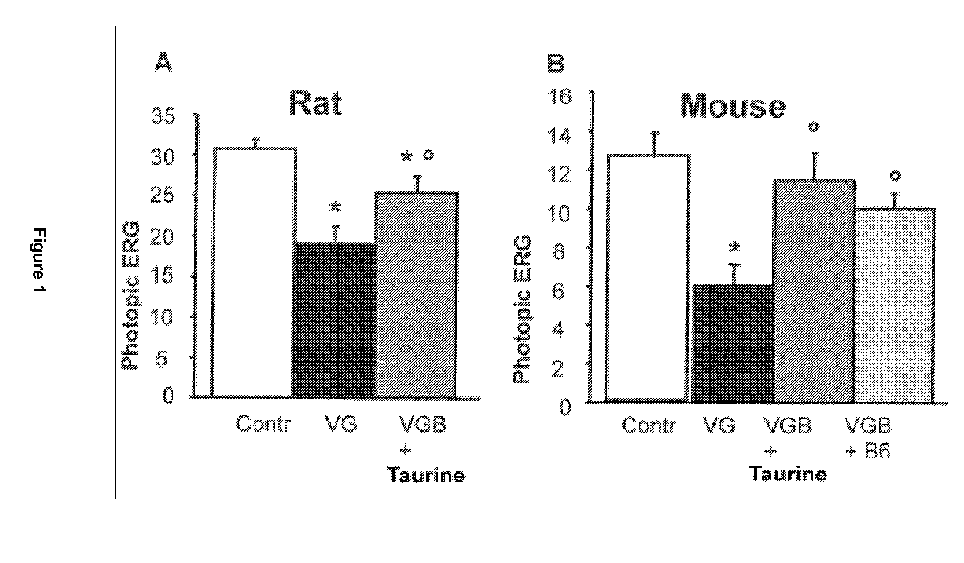

**Figure 1** **illustrates the neuroprotection by taurine or vitamin B6 against the side effects caused by the extracellular GABA-inducer vigabatrin.** When rats (Figure 1A) and mice (Figure 1 B) were treated with vigabatrin, a supplementation with taurine or vitamin B6 prevented or partially suppressed the decrease in amplitude of the photopic electroretinogram (ERG) observed under vigabatrin alone. The differences in the photopic ERG amplitudes were statistically significant between the control and vigabatrin treated animals (VGB: * p<0.05). By contrast, in mice, the differences between control animals and animals treated with vigabatrin plus taurine or vigabatrin plus vitamin B6 were no longer significant. In rats, the difference remained significant with the control group (VGB+ taurine: * p<0.05). However, in both mice and rats, animals receiving vigabatrin plus taurine had smaller decrease in photopic ERG amplitude than animals treated with vigabatrin alone; the differences were statistically significant (VGB+ taurine, ° p<0.05).. Similarly, mice receiving vitamin B6 supplementation in addition to vigabatrin had smaller decrease in photopic ERG than with vigabatrin alone and the difference was statistically significant (VGB+ B6, ° p<0.05).

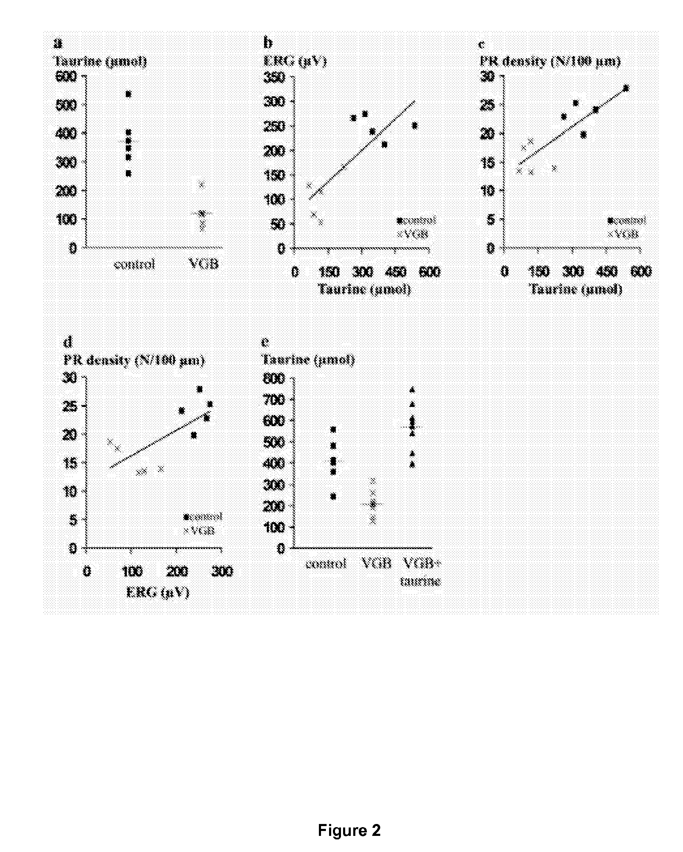

**Figure 2** **illustrates the correlation between taurine plasma levels and the irreversible visual effects of vigabatrin.** A) Taurine plasma levels in control and vigabatrin-treated animals in each group. B) Correlation between taurine plasma levels and the photopic ERG amplitude in VGB-treated control rats. The correlation factor between these two factors is equal to 0.769 (p=0.0093%, n=10). C) Correlation between the taurine plasma levels and the density of inner/outer cone photoreceptor segments. The correlation factor between these two parameters is equal to 0.818 (p=0.0038%, n=10). D) Correlation between the photopic ERG amplitudes and the density of inner/outer cone photoreceptor segments. The correlation factor between these two factors is equal to 0.703 (p=0.0023%, n=10). E) Recovery of taurine plasma levels by taurine supplementation in VGB-treated rats. In A and E, the horizontal line represents the mean value.

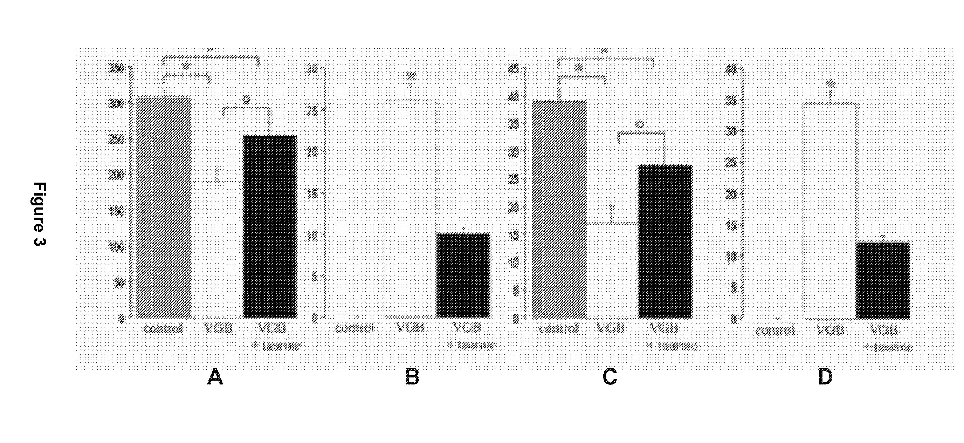

**Figure 3** **illustrates the prevention by taurine supplementation of all features of vigabatrin-induced irreversible visual effects in rats.** Quantification of photopic ERG amplitude (3A), length of retinal areas with displaced photoreceptor nuclei (3B), density of cone inner/outer segments (3C) and areas with increased GFAP expression (3D) in control rats (s.e.m., n=6), in the VGB-treated animals with or without taurine supplementation (VGB, n=7; VGB + taurine, n=7, s.e.m.). The scale bar represents 50µm (IPL: inner plexiform layer).

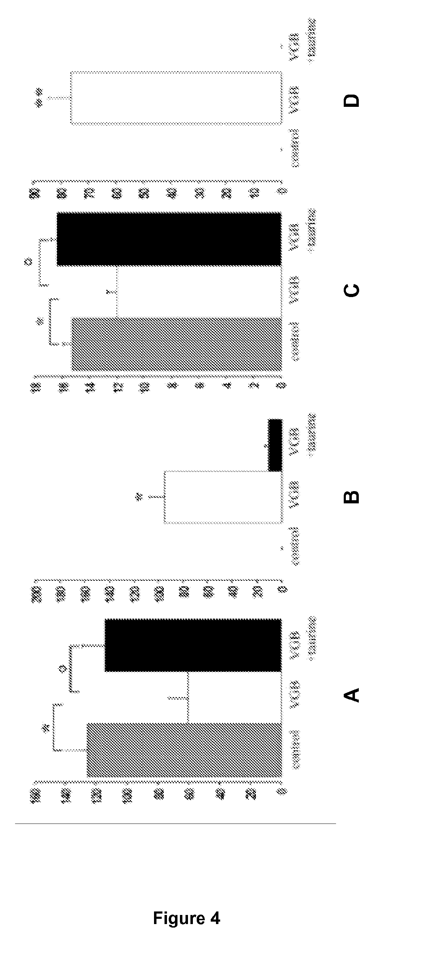

**Figure 4** **illustrates the prevention by taurine supplementation of all features of vigabatrin-irreversible visual effects in mice.** Quantification of photopic ERG amplitude (4A), length of retinal areas with displaced photoreceptor nuclei (4B), density of cone inner/outer segments (4C) and areas with bipolar cell sprouting into the ONL (4D) in control mice (s.e.m., n=5), in VGB-treated animals with or without taurine supplementation (VGB, s.e.m., n= 7; VGB + taurine, s.e.m., n= 7). The scale bar represents 50µm (IPL: inner plexiform layer).

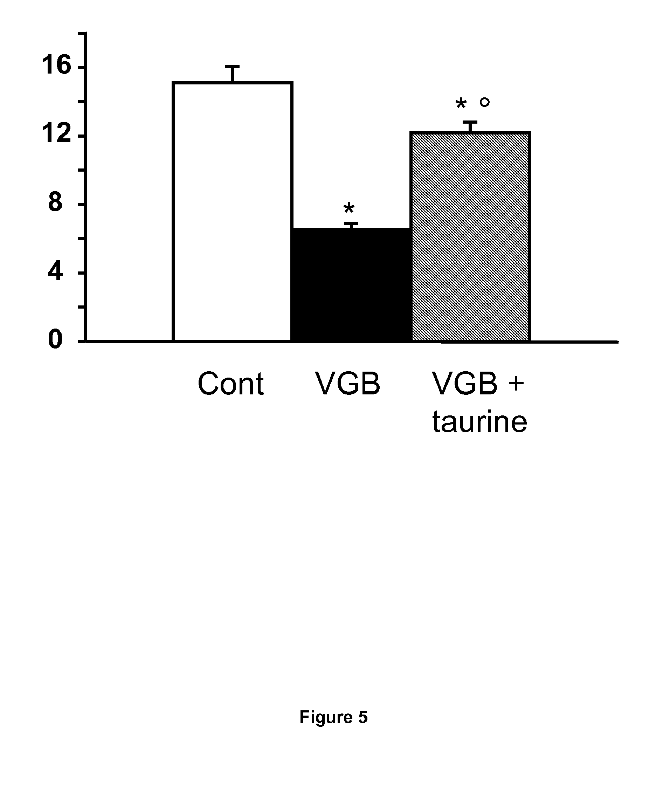

**Figure 5** **illustrates the effect of VGB and taurine on the degeneration of retinal ganglion cells.** Quantification of Brn3A-positive retinal ganglion cells in retinal sections of control animals (Cont), vigabatrin-treated rats (VGB) and vigabatrin-treated rats supplemented with taurine (VGB + taurine). The differences between control animals and the two other group were statistically significant (*) but the difference between the two groups of VGB-treated animals with or without taurine supplementation was also significant. (s.e.m., n=10).

### EXAMPLE 1:

### Material & Methods

As described previously (Duboc et al., 2004), Wistar rats Rj Wi IOPS Han and Balb c mice were purchased from Janvier Breeding Center (Le Genest St Isle, France) at 7 weeks. VGB was dissolved in 0.9 % NaCl at a concentration of 125 mg/ml. It was administered at 40 mg (125 mg/ml, 0.32 ml) by daily intraperitoneal injection for 45 or 65 days and at 3 mg (60 mg/ml, 0.05 ml) to mice for 29 days (daily dose of about 200 mg/kg in rats and 150 mg/kg in mice). Vitamin B6 (active form) was administered at a dose of 1 mM/kg (solution 154,46 mg/ml) whereas taurine was provided in the drinking water at 0,1 mol/l.

ERG were recorded as described previously after the last VGB injection (Duboc et al., 2004). Anesthesia was induced by an intramuscular injection of a solution containing ketamine (100 mg/kg) and xylazine (10 mg/kg). After 10 min adaptation to a 2.5 cdm⁻² light intensity, ten recordings were obtained with flash intensity of 10 cdsm⁻² at an interstimulus interval 30s and subsequently averaged. These photopic ERG measurements were performed after 59 days in rats and 29 days in mice.

After 65 days of injection, rats were anesthetized for blood sampling. Anesthesia was induced as described above. Under anesthesia an indwelling catheter was placed in the carotid artery. This method allowed obtaining a single, large, good quality blood sample. A 1 mL blood sampling was taken for each rat in a heparinized blood sampler, immediately centrifuged and frozen at -80°C. Automated amino acid ion chromatography (Pasteur Cerba, France) was used for the quantitative determination of total and free amino acids in the samples.

### Results

**Taurine deficiency induced by the extracelular GABA inducer Vigabatrin:** In Table 1, the amino acid concentrations measured on blood samples for both vigabatrin-treated and control albino animals are presented. It indicates that concentration differences ranging from 7 to 32 % were not statistically significant for most amino acids thréonine, sérine, valine, citrulline, alanine, glycine, proline, glutamine, isoleucine, leucine, tyrosine, phenylalanine, arginine, histidine, lysine, ornithyne. As previously reported the glutamic acid was greatly reduced by 56% in the blood sample and this difference was statistically significant. However, the greatest change was observed for taurine which was decreased by 67% in vigabatrin-treated animals from 373.4 to 122.2 µM. This difference was statistically significant (p<0.05). A significant difference (22%) was also observed for methionine. These observations indicated that taurine and methionine concentrations are significantly reduced by the vigabatrin treatment.

**Table 1: Blood concentrations for amino acids in albino rats treated with vigabatrin (VGB) or not treated (control). Measures are provided in µM (n=5, s.e.m.).**

| | **Control** | **VGB** |
|---|---|---|
| Taurine | 373,4 ± 46,7 | 122,2 ± 26,6 * |
| Thréonine | 194,2 ± 30,8 | 130,6 ± 7,0 |
| Sérine | 203,8 ± 28,7 | 169,4 ± 16,1 |
| Acide glutamique | 217,4 ± 56,6 | 94,8 ± 15,6 * |
| Valine | 167, 4 ± 14,8 | 146 ±8,9 |
| Citrulline | 54 ± 9,7 | 45,6 ± 4,2 |
| Alanine | 303,8 ± 32,3 | 270,4 ± 29,5 |
| Glycine | 190,6 ± 30,1 | 142,8 ± 17,6 |
| Proline | 155 ± 21,0 | 110,6 ± 20,1 |
| Glutamine | 375,6 ± 40,7 | 349 ± 13,4 |
| Methionine | 58 ± 4,7 | 45 ± 1,4 * |
| Isoleucine | 79,8 ± 8,0 | 68,2 ± 4,5 |
| Leucine | 152,6 ± 17,6 | 123,2 ± 11,5 |
| Tyrosine | 63,2 ± 6,8 | 51,6 ± 4,5 |
| Phenylalanine | 54,2 ± 6, 9 | 42,8 ± 1,0 |
| Arginine | 130 ± 39,0 | 111,8 ± 15,1 |
| Histidine | 75,8 ± 5,4 | 63,2 ± 2,9 |
| Lysine | 307,2 ± 23,8 | 260,4 ± 32,3 |
| Ornithine | 78.6 ± 33.0 | 57.6 ±10.6 |

**Prevention and treatment of the undesirable side effects caused by the extracellular GABA inducer Vigabatrin by taurine and a taurine-like substance :** To investigate whether taurine supplementation could prevent the vigabatrin-induced irreversible visual effects, animals were treated with vigabatrin alone or vigabatrin plus a taurine supplementation. Vigabatrin alone induced a decrease in the photopic electroretinogram (ERG) measurement as previously described. By contrast, in mice, the difference was no longer statistically significant between controls and vigabatrin plus taurine supplementation or vigabatrin plus vitamin B6 supplementation (Fig. 1 B). In rats, although the photopic ERG amplitude decreased less than in vigabatrin alone, the difference remained statistically significant with the group of control animals (Fig1A). However, in all species, the difference between vigabatrin and vigabatrin plus taurine supplementation was statistically significant in treated animals (Fig.1). Similarly, the supplementation with vitamin B6 also reduced the decrease in photopic ERG amplitude and the difference between the mouse group of vigabatrin alone and the group receiving vigabatrin plus vitamin B6 supplementation was statistically significant (Fig.1B). These results are consistent with the hypothesis that vigabatrin-induced decrease in taurine concentration could produce the irreversible visual effects of vigabatrin.

### EXAMPLE 2:

### Material & methods:

**Animal treatments:** As described previously (Duboc, A., et al. Vigabatrin, the GABA-transaminase inhibitor, damages cone photoreceptors in rats. Annals of neurology Vol. 55, 695-705 (2004).), Wistar rats Rj Wi IOPS Han or BALB/c mice were purchased from Janvier (Le Genest-St-lsle, France) at between six and seven weeks of age. VGB dissolved in 0.9% NaCl was administered at 40mg (125 mg/ml, 0.32ml) to rats by daily intraperitoneal injection for 45 or 65 days and at 3mg (60mg/ml, 0.05ml) to mice for 29 days. These daily doses (rats:200mg/kg; mice:150mg/kg) are in-line with those described for the treatment of epilepsy in animals (Andre, V., Ferrandon, A., Marescaux, C. & Nehlig, A. Vigabatrin protects against hippocampal damage but is not antiepileptogenic in the lithium-pilocarpine model of temporal lobe epilepsy. Epilepsy research Vol. 47, 99-117 (2001)) or in humans (adult patients: 1-6mg/kg; children:50-75mg/kg; or infants: 100-400 mg/kg) (Chiron, C., Dumas, C., Jambaque, I., Mumford, J. & Dulac, O. Randomized trial comparing vigabatrin and hydrocortisone in infantile spasms due to tuberous sclerosis. Epilepsy research Vol. 26, 389-395 (1997); Lux, A.L., et al. The United Kingdom Infantile Spasms Study comparing vigabatrin with prednisolone or tetracosactide at 14 days: a multicentre, randomised controlled trial. Lancet Vol. 364, 1773-1778 (2004); Aicardi, J., Mumford, J.P., Dumas, C. & Wood, S. Vigabatrin as initial therapy for infantile spasms: a European retrospective survey. Sabril IS Investigator and Peer Review Groups. Epilepsia Vol. 37, 638-642 (1996)). Taurine supplementation was given in drinking water at a concentration of 0.1M. Light intensity in the animal cages ranged between 125 and 130 lux for rats and between 70 and 85 lux for mice.

**Electroretinogram (ERG):** Photopic ERGs were recorded after the last VGB injection, as described previously (Duboc, A., et al. Vigabatrin, the GABA-transaminase inhibitor, damages cone photoreceptors in rats. Annals of neurology Vol. 55, 695-705 (2004).). Anesthesia was induced by intraperitoneal injection (0.8 to 1.2 ml/kg) of a solution containing ketamine (40 mg/ml) and xylazine (4 mg/ml Rompum). Animals were light-adapted for 10 minutes with a background light of 25 cdm⁻². Light flashes were then applied on this background light; the light intensity of the flash was 10 cdsm⁻² for the mouse experiments and for the rats maintained in darkness (Figs. 2, 6) or 25 cdsm⁻² for other experiments (Figs 3, 5). Ten recordings were averaged with an interstimulus interval of 30s.

**Histology:** Eye cups were fixed overnight at 4°C in 4% (wt/vol) paraformaldehyde in phosphate buffered saline (PBS; 0.01 M, pH 7.4). The tissue was cryoprotected in successive solutions of PBS containing 10%, 20% and 30% sucrose at 4°C, oriented along the dorso-ventral axis and embedded in OCT (Labonord, Villeneuve d'Ascq, France). Retinal sections (8-10µm thickness) were permeabilised for five minutes in PBS containing 0.1% Triton X-100 (Sigma, St. Louis, MO), rinsed, and incubated in PBS containing 1% bovine serum albumin (Eurobio, Les-Ulis, France), 0.1% Tween 20 (Sigma), and 0.1% sodium azide (Merck, Fontenay-Sous-Bois, France) for two hours at room temperature. The primary antibody added to the solution was incubated for two hours at room temperature. Polyclonal antibodies were directed against VGluT1 (1:2000, Chemicon), rabbit GFAP (1/400, Dako, USA) and mouse cone arrestin (Zhu, X., et al. Mouse cone arrestin expression pattern: light induced translocation in cone photoreceptors. Molecular vision Vol. 8, 462-471 (2002)) (Luminaire junior, LUMIj, 1:20,000). Monoclonal antibodies were directed against Goα (1:2000, Chemicon). Sections were rinsed and then incubated with the secondary antibody, goat anti-rabbit IgG or rabbit anti-mouse IgG conjugated to either Alexa TM594 or Alexa TM488 (1:500, Molecular Probes, Eugene, OR) for two hours. Inner/outer segments of cone photoreceptors were stained with a peanut lectin (PNA, Sigma) during 12 hours of incubation at 4°C. The dye, diamidiphenyl-indole (DAPI), was added during the final incubation period. Sections were rinsed, mounted with Fluorsave reagent (Calbiochem, San Diego, CA) and viewed with a Leica microscope (LEICA DM 5000B) equipped with a Ropper scientific camera (Photometrics cool SNAP TM FX).

For quantification, vertical sections along the dorso-ventral axis were selected at the optic nerve. Following DAPI nuclear staining, the lengths of disorganised retinal areas were measured; GFAP immunostaining was used for detection and quantificationof retinal areas with reactive gliosis. The cone photoreceptor density was calculated following cone arrestin immunostaining (rats) or PNA labelling (mice) to visualise the inner/outer segments of cone photoreceptors; areas with disorganised retinal layering were excluded. Areas with sprouting bipolar cells were quantified following immunolabelling of these cells with the antibody directed against the protein Goα.

### Results:

Taurine deficiency is known to induce photoreceptor degeneration in mice (Rascher et al., 2004), rats (Lake and Malik, 1987), cats (Leon et al., 1995) and monkeys (Imaki et al., 1987). Although taurine levels had instead been reported to be unchanged or increased in vigabatrin-treated animals (Halonen et al., 1990; Neal and Shah, 1990) and patients (Halonen et al., 1988; Pitkanen et al., 1988), we examined the potential role of taurine in VGB irreversible visual effects. Plasma amino-acid levels were measured in control animals and VGB-injected rats treated for 65 days. The differences in concentrations for most amino acids (threonine, serine, valine, citrulline, alanine, glycine, proline, glutamine, isoleucine, leucine, tyrosine, phenylalanine, arginine, histidine, lysine, and ornithine) were not statistically different between the two groups of animals. By contrast, the plasma glutamic acid concentrations were much lower (by 56%) in VGB-treated animals than in control animals, consistent with previous reports (Bernasconi, R., et al. Gamma-vinyl GABA: comparison of neurochemical and anticonvulsant effects in mice. J Neural Transm Vol. 72, 213-233 (1988)); this difference was statistically significant (control : 217.4±56.6 µM; VGB: 94.8±15.6 µM s.e.m., n=5), as already shown in Table 1 of example 1. However, the greatest difference was observed for taurine. Taurine levels were by 67% lower in VGB-treated animals (122.2±26.6 µM) than in control animals (373.4±46.7 µM) (Fig. 2A, p<0.05, n=5, s.e.m.). A significant difference (22% reduction) was also observed for methionine. To determine if this decrease in taurine level is correlated to visual loss, both ERG amplitudes (r =0.769, p=0.0093%) and cone densities (r=0.818, p=0.0038%) were plotted against taurine levels in control animals and VGB-treated rats (Fig. 2B, 2C). Taurine levels were highly correlated to both ERG amplitudes and cone densities; these two factors were also correlated with each other (Fig. 2D, r =0.703, p=0.0023%). Thus, the low level of circulating taurine may directly contribute to retinal VGB toxicity in rats.

Taurine is mainly obtained from nutrition; thus, we supplemented the drinking water of one group of VGB-treated rats with taurine (0.1 M) (n=7). The plasma levels of taurine in these animals increased above those of control animals and above those of VGB-treated animals without taurine supplementation (Fig. 2E). The photopic ERG amplitude was greater in VGB-treated animals receiving taurine supplementation than in VGB-treated animals without taurine supplementation (n=7) (Fig. 3A, ° p<0.01), but remained lower than in control animals (n=5) (Fig. 3A, * p<0.05). Similarly, the cone segment density was greater in the VGB-treated group with taurine supplementation than in animals without supplementation (Fig. 3B, ° p<0.05), but remained lower than that of the control group (Fig. 3B, * p<0.001). Furthermore, the VGB-induced increase in GFAP immunostaining was less extensive in animals receiving taurine supplementation than in animals without supplementation (Fig. 3C, ° p<0.001). These findings show that VGB-induced retinal damage can be attenuated by taurine supplementation in rats.

To determine whether taurine can prevent VGB irreversible visual effects in another species, mice were administered VGB with (n=7) or without taurine supplementation (n=7) for 29-30 days. As in rats, VGB-treated mice had significantly lower photopic ERG amplitude than the control group (n=5) (Fig. 4A, * p<0.01). Taurine supplementation suppressed this decrease in photopic ERG amplitude such that the difference between these mice and the control group was not statistically different. The difference between the two VGB-treated groups with or without taurine supplementation was statistically significant (Fig. 4A, ° p<0.05). Quantification of the disorganisation of the outer nuclear layer in the two VGB-treated groups showed that mice with taurine supplementation had smaller areas of retinal disorganisation than mice without supplementation (Fig. 4B, ° p<0.001). Quantification of cone outer and inner segments following lectin staining of their extracellular matrix indicated that VGB treatment resulted in lower densities (Fig. 4C, * p<0.001). Taurine supplementation prevented this decrease in cone density (Fig. 4D, ° p<0.001). Finally, the sprouting of bipolar cell dendrites, observed in VGB-treated mice, was completely suppressed by the taurine supplementation. These results indicate that taurine supplementation also reduces features of VGB toxicity in mice.

### EXAMPLE 3:

### Material & methods:

Blood samples from patients with epilepsy were collected in haemolysis tubes containing heparin (14 IU/ml) and centrifuged (2200g, 15min). Plasma amino-acid analysis was performed by ion-exchange chromatography with nihydrin detection using a JEOL AMINOTAC analyser.

### Results:

We then evaluated whether these findings have any clinical relevance in patients with epilepsy. Plasma taurine levels were reviewed retrospectively in patients presenting infantile spasms, who had received VGB treatment for at least six months in Necker Hospitals for the last 3 years. Plasma amino-acid levels are routinely measured in patients with infantile spasms as part of their etiological work-up, particularly when brain RMI is negative. Data were available in six patients (Table 2 hereunder). Five patients had a taurine level below normal values for infants of a similar age; taurine was undetectable in two patients. For one of these two patients (patient 2), the taurine level was measured before starting a 15 month period of VGB therapy and this taurine level was normal. In patient 6, the taurine level was in the lower part of the normal range, but this measurement could have been overestimated by the delayed blood centrifugation. Thus, taurine levels seem to be decreased in VGB-treated epileptic infants.

**Table 2:**

| **Patients** | **1** | **2** | | **3** | **4** | **5** | **6** |
|---|---|---|---|---|---|---|---|
| **Age at VGB onset** | 6m | 9m | | 7m | 3m | 3m | 6m |
| **Age at taurine measurement** | 2.5y | 8.5m | 2y | 3y | 1.2y | 9m | 2.1y |
| **Taurine level (µmoles/l)** | **und*** | **75** | **und** | **26** | **20** | **38** | **52** |
| **Normal values (µmoles/l ± SD) at corresponding age** | 70±22 | 81±35 | 70±22 | 70±22 | 76±36 | 81±35 | 70±22 |
| **Delay between blood sampling and plasma preparation** | <2h | <2h | <2h | <2h | <2h | 48h | 72h |

| | | | | | | | |
|---|---|---|---|---|---|---|---|
| und*: undetectable SD: Standard Deviation | | | | | | | |

### EXAMPLE 4:

### Methods:

**Animal treatments:** Breeds of Wistar rats Rj Wi IOPS Han were purchased from Janvier (Le Genest-St-lsle, France). VGB dissolved in 0.9% NaCl was administered at 0.6mg/jour (6 mg/ml, 0.1ml) to rats by daily intraperitoneal injection for 25 days from age 4 days. These daily doses (50mg/kg for rats of 12g) are in-line with those described for the treatment of epilepsy (adult patients: 1-6mg/kg; children:50-75mg/kg; or infants: 100-150 mg/kg) (Chiron C. et al. 1997; Lux AL. et al. 2004; Aicardi J. et al. 1996). Taurine supplementation was administered by intraperitoneal injections at a concentration of 5mg/day (50mg/ml dans 0.1ml).

**Histology:** Animals were anesthetized by intraperitoneal injection (0.8 to 1.2 ml/kg) of a solution containing ketamine (40 mg/ml) and xylazine (4 mg/ml Rompum). They were then perfused first with heaparin 1000Ul/ml in saline solution (0.9% NaCl), then with fixative containing 4% Paraformaldehyde (Merck chemicals ref. : 100496 ) and 0.2% glutaraldehyde (fluka 25%). Eye cups were isolated and fixed overnight at 4°C in 4% (wt/vol) paraformaldehyde in phosphate buffered saline (PBS; 0.01 M, pH 7.4). The tissue was cryoprotected in successive solutions of PBS containing 10%, 20% and 30% sucrose at 4°C, oriented along the dorso-ventral axis and embedded in OCT (Labonord, Villeneuve d'Ascq, France). Retinal sections (8-10µm thickness) were permeabilised for five minutes in PBS containing 0.1% Triton X-1 00 (Sigma, St. Louis, MO), rinsed, and incubated in PBS containing 1% bovine serum albumin (Eurobio, Les-Ulis, France), 0.1% Tween 20 (Sigma), and 0.1% sodium azide (Merck, Fontenay-Sous-Bois, France) for two hours at room temperature. The primary Brn3A monoclonal antibody (Chemicon) was added to the solution and incubated for two hours at room temperature. Sections were rinsed and then incubated with the secondary antibody, rabbit anti-mouse lgG conjugated to either Alexa TM594 (1:500, Molecular Probes) for two hours. Sections were rinsed, mounted with Fluorsave reagent (Calbiochem) and viewed with a Leica microscope (LEICA DM 5000B) equipped with a Ropper scientific camera (Photometrics cool SNAP TM FX).

For quantification, vertical sections along the dorso-ventral axis were selected at the optic nerve. Brn3A -positive retinal ganglion cells were quantified along the complete retinal section.

**Statistical analysis:** Statistical analysis of the results was performed by a one-way analysis of variance with the Student-Newman Keuls test (Sigmastat).

### Results:

Figure 5 illustrates the effect of VGB and taurine on the degeneration of retinal ganglion cells. When young rats were treated with VGB, the number of retinal ganglion cells was decreased by 56% (control: 15,06 ± 0.93; VGB: 6.56 ± 0.32, s.e.m., n= 10). The difference was statistically significant (p<0.001). By contrast, when rats received taurine supplementation, the number of retinal ganglion cells was less decreased (VGB+taurine: 12,13 ± 0.62, s.e.m., n= 10). The difference with the group of VGB-treated rats was statistically significant (p<0.001). However the taurine supplementation did not completely rescue retinal ganglion cells, the difference with the control group remained significant (p<0.005). These results indicated that taurine supplementation can suppress the VGB-induced degeneration of retinal ganglion cells.

### REFERENCES:

Aicardi J, Mumford JP, Dumas C, Wood S. Vigabatrin as initial therapy for infantile spasms: a European retrospective survey. Sabril IS Investigator and Peer Review Groups. Epilepsia. 1996;37:638-642
Baram TZ, Mitchell WG, Tournay A et al. High-dose corticotropin (ACTH) versus prednisone for infantile spasms: a prospective, randomized, blinded study. Pediatrics. 1996;97:375-379
Ben-Menachem E, Dulac O, Chiron C. Vigabatrin. In: Epilepsy: a comprehensive textbook. Editors Jerome Engel Jr and Timothy A Pedley. Lippincott Williams & Wilkins, Philadelphia.. 2008;Second edition:1683-1693.
Buncic JR, Westall CA, Panton CM, Munn JR, MacKeen LD, Logan WJ. Characteristic retinal atrophy with secondary "inverse" optic atrophy identifies vigabatrin toxicity in children.Ophtalmology 2004; 111:1935-42
Butler WH, Ford GP, Newberne JW. A study of the effects of vigabatrin on the central nervous system and retina of Sprague Dawley and Lister-Hooded rats. Toxicologic pathology. 1987;15:143-148
Chiron C, Dumas C, Jambaque I et al. Randomized trial comparing vigabatrin and hydrocortisone in infantile spasms due to tuberous sclerosis. Epilepsy research. 1997;26:389-395
Cubells JF, Blanchard JS, Makman MH. The effects of in vivo inactivation of GABA-transaminase and glutamic acid decarboxylase on levels of GABA in the rat retina. Brain research. 1987;419:208-215
Duboc A, Hanoteau N, Simonutti M et al. Vigabatrin, the GABA-transaminase inhibitor, damages cone photoreceptors in rats. Annals of neurology. 2004;55:695-705
Duboc A, Hanoteau N, Simonutti M, Rudolf G, Nehlig A, Sahel JA, Picaud S (2004) Vigabatrin, the GABA-transaminase inhibitor, damages cone photoreceptors in rats. Ann Neurol 55:695-705.
Dulac O, Dalla Bernardina B, Chiron C. West syndrome. In: Epilepsy: a comprehensive textbook. Editors Jerome Engel Jr and Timothy A Pedley. Lippincott Williams & Wilkins, Philadelphia.. 2008; Second edition:2329-2335
Gerasimov MR, Dewey SL. Gamma-vinyl gamma-aminobutyric acid attenuates the synergistic elevations of nucleus accumbens dopamine produced by a cocaine/heroin (speedball) challenge. Eur J Pharmacol. 1999;380:1-4
Halonen T, Lehtinen M, Pitkanen A, Ylinen A, Riekkinen PJ (1988) Inhibitory and excitatory amino acids in CSF of patients suffering from complex partial seizures during chronic treatment with gamma-vinyl GABA (vigabatrin). Epilepsy Res 2:246-252.
Halonen T, Pitkanen A, Riekkinen PJ (1990) Administration of vigabatrin (gamma-vinyl-gamma-aminobutyric acid) affects the levels of both inhibitory and excitatory amino acids in rat cerebrospinal fluid. J Neurochem 55:1870-1874.
Hilton EJ, Cubbidge RP, Hosking SL et al. Patients treated with vigabatrin exhibit central visual function loss. Epilepsia. 2002;43:1351-1359
Hrachovy RA, Frost JD, Jr., Glaze DG. High-dose, long-duration versus lo-dose, short-duration corticotropin therapy for infantile spasms. The Journal of pediatrics. 1994;124:803-806
Imaki H, Moretz R, Wisniewski H, Neuringer M, Sturman J (1987) Retinal degeneration in 3-month-old rhesus monkey infants fed a taurine-free human infant formula. J Neurosci Res 18:602-614.
Izumi Y, lshikawa M, Benz AM et al. Acute vigabatrin retinotoxicity in albino rats depends on light but not GABA. Epilepsia. 2004;45:1043-1048
Johnson MA, Krauss GL, Miller NR et al. Visual function loss from vigabatrin: effect of stopping the drug. Neurology. 2000;55:40-45
Lake N, Malik N (1987) Retinal morphology in rats treated with a taurine transport antagonist. Exp Eye Res 44:331-346.
Leon A, Levick WR, Sarossy MG (1995) Lesion topography and new histological features in feline taurine deficiency retinopathy. Exp Eye Res 61:731-741.
Lux AL, Edwards SW, Hancock E et al. The United Kingdom Infantile Spasms Study (UKISS) comparing hormone treatment with vigabatrin on developmental and epilepsy outcomes to age 14 months: a multicentre randomised trial. Lancet Neurol. 2005;4:712-717
Lux AL, Edwards SW, Hancock E et al. The United Kingdom Infantile Spasms Study comparing vigabatrin with prednisolone or tetracosactide at 14 days: a multicentre, randomised controlled trial. Lancet. 2004;364:1773-1778
McDonagh J, Stephen LJ, Dolan FM et al. Peripheral retinal dysfunction in patients taking vigabatrin. Neurology. 2003;61:1690-1694
Miller NR, Johnson MA, Paul SR et al. Visual dysfunction in patients receiving vigabatrin: clinical and electrophysiologic findings. Neurology. 1999;53:2082-2087
Neal MJ, Cunningham JR, Shah MA, Yazulla S. lmmunocytochemical evidence that vigabatrin in rats causes GABA accumulation in glial cells of the retina. Neuroscience letters. 1989;98:29-32
Neal MJ, Shah MA (1990) Development of tolerance to the effects of vigabatrin (gamma-vinyl-GABA) on GABA release from rat cerebral cortex, spinal cord and retina. Br J Pharmacol 100:324-328.
Pitkanen A, Matilainen R, Ruutiainen T, Lehtinen M, Riekkinen P (1988) Effect of vigabatrin (gamma-vinyl GABA) on amino acid levels in CSF of epileptic patients. J Neurol Neurosurg Psychiatry 51:1395-1400.
Rascher K, Servos G, Berthold G, Hartwig HG, Warskulat U, Heller-Stilb B, Haussinger D (2004) Light deprivation slows but does not prevent the loss of photoreceptors in taurine transporter knockout mice. Vision Res 44:2091-2100.
Ravindran J, Blumbergs P, Crompton J, Pietris G, Waddy H. Visual field loss associated with vigabatrin: pathological correlations. J Neurol. Neurosurg Psychiatry 2001; 70:787-9
Sills GJ, Patsalos PN, Butler E et al. Visual field constriction: accumulation of vigabatrin but not tiagabine in the retina. Neurology. 2001;57:196-200
Snead OC, 3rd, Benton JW, Myers GJ. ACTH and prednisone in childhood seizure disorders. Neurology. 1983;33:966-970
Stromberg MF, Mackler SA, Volpicelli JR et al. The effect of gamma-vinyl-GABA on the consumption of concurrently available oral cocaine and ethanol in the rat. Pharmacol Biochem Behav. 2001;68:291-299
Wang QP, Jammoul F, Duboc A et al. Treatment of epilepsy: the GABA-transaminase inhibitor, vigabatrin, induces neuronal plasticity in the mouse retina. Eur J Neurosci. 2008;27:2177-2187

## Claims

1. A substance selected from the group consisting of taurine, a taurine precursor, a taurine metabolite, a taurine derivative, a taurine analog and a substance required for the taurine biosynthesis for the prevention of the irreversible visual effects of vigabatrin.

2. The substance according to claim 1, wherein said taurine precursor is selected from the group consisting of cysteine, cystathionine, homocysteine, S-adenosylhomocysteine, serine, N-acetyl-cysteine, glutathione, N-formylmethionine, S-adenosylmethionine, betaine and methionine.

3. The substance according to claim 1 wherein said taurine metabolite is selected from the group consisting of hypotaurine, thiotaurine, taurocholate.

4. The substance according to claim 1 wherein said taurine derivative is selected from the group consisting of of acetylhomotaurinate, and piperidino-, benzamido-, phthalimido- or phenylsuccinylimido taurine derivatives taurolidine, taurultam and taurinamide, chlorohydrate-N-isopropylamide-2-(1-phenylethyl)aminoethanesulfonic acid.

5. The substance according to claim 1, wherein said taurine analog is selected from the group consisting of (+/-)piperidine-3-sulfonic acid (PSA), 2-aminoethylphosphonic acid (AEP), (+/-)2-acetylaminocyclohexane sulfonic acid (ATAHS), 2-aminobenzenesulfonate (ANSA), hypotaurine,. ± trans-2-aminocyclopentanesulfonic acid (TAPS) 8-tétrahydroquinoléine sulfonic acid (THQS), N-2-hydroxyethylpiperazine-N'-2-ethane sulphonic acid (HEPES), beta-alanine, glycine, guanidinoethylsulfate (GES), 3-acétamido-1-propanesulfonic acid (acamprosate).

6. The substance according to claim 1, wherein the substance required for the taurine biosynthesis is selected from the group consisting of vitamin B6, vitamin B12, folic acid, riboflavin, pyridoxine, niacin, thiamine, and pantothenic acid.

7. A pharmaceutical composition for the prevention of the irreversible visual effects of vigabatrin which comprises a substance according to any one claims 1 to 6, and optionally one or more pharmaceutically acceptable excipients

8. A pharmaceutical composition for the treatment of convulsive disorders which comprises (i) a first active ingredient which is vigabatrin; and (ii) a second active ingredient selected from the group consisting of taurine, a taurine precursor, a taurine metabolite, a taurine derivative, a taurine analog and a substance required for the taurine biosynthesis and (iii) optionally one or more pharmaceutically acceptable excipients.

9. The pharmaceutical composition according to claim 8 wherein said convulsive disorders is selected from the group consisting of epilepsy, tuberous sclerosis, infantile spasms and convulsive disorders affecting patients undergoing a drug addiction, including a drug addiction to heroin or cocaine, ethanol.
